# EUROPÄISCHE PATENTSCHRIFT

(11) **EP 1 042 261 B1**
(45) Veröffentlichungstag und Bekanntmachung des Hinweises auf die Patenterteilung: **21.05.2003**
(21) Anmeldenummer: 98912480.5
(22) Anmeldetag: 16.03.1998
(51) Int. Cl.: C07C 29/78, C07C 27/02, C07C 67/03, C07C 67/02, C07C 69/52, C07C 29/149

(54) **VERFAHREN ZUR HERSTELLUNG VON UNGESÄTTIGTEN FETTALKOHOLEN MIT VERBESSERTEM KÄLTEVERHALTEN**
METHOD FOR THE PRODUCTION OF NON-SATURATED FATTY ALCOHOLS WITH IMPROVED LOW-TEMPERATURE BEHAVIOUR
PROCEDE DE PREPARATION D'ALCOOLS GRAS INSATURES A COMPORTEMENT A FROID AMELIORE

(30) Priorität: 25.03.1997 DE 19712506
(43) Veröffentlichungstag der Anmeldung: 11.10.2000
(73) Patentinhaber: Cognis Deutschland GmbH & Co. KG, 40589 Düsseldorf (DE)
(72) Erfinder: NITSCHE, Michael, D-42655 Solingen (DE); HECK, Stephan, D-50259 Pulheim (DE); LINDEMANN, Manfred, D-42719 Solingen (DE); JOHANNISBAUER, Wilhelm, D-40699 Erkrath (DE); BRENDLER, Lutz, D-06217 Merseburg (DE); KLEIN, Norbert, D-40822 Mettmann (DE)
(74) Vertreter: Fabry, Bernd, Dr.
(86) Internationale Anmeldenummer: EP9801512
(87) Internationale Veröffentlichungsnummer: WO98042646

(56) Entgegenhaltungen:
- EP-A- 0 113 798
- DE-A- 4 425 180
- DE-C- 4 422 858

## Beschreibung

### Gebiet der Erfindung

Die Erfindung betrifft ein Verfahren zur Herstellung von ungesättigten Fettalkoholen mit erhöhter lodzahl und vermindertem Trübungspunkt durch fraktionierte Kristallisation.

### Stand der Technik

Fettstoffe, insbesondere ungesättigte Fettalkohole, stellen wichtige Zwischenprodukte beispielsweise für die Herstellung von Tensiden und kosmetischen Produkten dar. Eine Übersicht von U.Ploog zu diesem Thema findet sich in **Seifen-Fette-Öle-Wachse** 109, 225 (1983).

Die Herstellung der ungesättigten Fettalkohole gelingt nicht auf Basis petrochemischer Rohstoffe und Verfahren, man hat vielmehr von mehr oder minder ungesättigten natürlichen Rohstoffen, vorzugsweise Fetten und Ölen sowie den daraus erhältlichen Fettsäuren bzw. Methylestem auszugehen, welche dann in Gegenwart von chrom- und/oder zinkhaltingen Mischoxidkatalysatoren unter Erhalt der Doppelbindung hydriert werden **[Ullmanns Enzyklopaedie der technischen Chemie, Verlag Chemie, Weinheim, 4.Aufl., Bd.11, S.436f]**. Die Herstellung ungesättigter Fettalkohole kann grundsätzlich auf drei Wegen erfolgen:
1. Fette und Öle werden einer Druckspaltung mit Wasser unterworfen. Nach Abtrennung des wäßrigen Glycerins werden Spaltfettsäuren erhalten, die Gemische gesättigter und ungesättigter Fettsäuren darstellen. Da eine gemeinsame Hydrierung dieser Säuren das Verhältnis gesättigter und ungesättigter Anteile nicht verschieben kann, werden auf diesem Wege nur Fettalkohole einer niedrigen lodzahl im Bereich von kleiner 80, vorzugsweise 50 bis 55 erhalten.
2. Eine destillative Trennung gesättigter und ungesättigter Fettsäuren gleicher Anzahl von Kohlenstoffatomen ist nur mit einem unverhältnismäßig hohen technischen Aufwand möglich. Abweichend von Verfahren 1 können die aus den Triglyceriden gewonnenen Spaltfettsäuren jedoch über den Weg der Umnetztrennung in einen überwiegend gesättigten und einen überwiegend ungesättigten Fettsäureschnitt überführt werden. Die Hydrierung des ungesättigten Fettsäureanteils - gegebenenfalls nach Veresterung mit Methanol - liefert technische Oleylalkohole eines lodzahlbereiches von etwa 80 bis 85, die in der Technik durch fraktionierte Destillation zu Produkten mit einer lodzahl von 90 bis 100 weiterverarbeitet werden.
3. Weiterhin ist es möglich, hochungesättigte Pflanzenöle einer Umesterung zu unterwerfen, bei der Methylester mit einem vergleichsweise geringen Anteil an gesättigten Homologen anfallen. Eine Umnetztrennung ist in diesem Fall weder möglich, noch erforderlich, da die Hydrierung unmittelbar hochungesättigte Fettalkohole mit einer lodzahl oberhalb von 100 liefert.

Die drei genannten Verfahren werden seit langem kommerziell zur Herstellung von ungesättigten Fettalkoholen genutzt, sind jedoch mit einer Reihe von Nachteilen verbunden: Die nach Verfahren 1 erhältlichen Produkte besitzen eine lodzahl unterhalb von 80 und sind wachsartig. Neben des unvorteilhaften Erstarrungspunktes weisen sie die Vorteile, die mit einer ungesättigten Struktur verbunden sind, naturgemäß nur anteilig auf. Üblicherweise kommen als Rohstoffe für das Verfahren 2 nur Fette und Öle mit einer lodzahl im Bereich von 40 bis 70 (z.B. Rindertalg, Schweineschmalz, Palmöl oder Palmstearin) in Frage. Die resultierenden Fettalkohole weisen eine lodzahl im Bereich von 90 bis 100 auf und kommen aufgrund ihres Eigenschaftsprofils am ehesten für eine technische Verwendung in Frage. Sie sind jedoch häufig weder hinsichtlich ihrer Farb- noch Geruchsqualität zufriedenstellend und weisen für viele Anwendungen einen ebenfalls unvorteilhaft hohen Erstarrungs- bzw. Trübungspunkt auf. Letzteres trifft im übrigen auch für ungesättigte Fettalkohole des gleichen lodzahlbereiches auf Basis beispielsweise neuen Sonnenblumenöls zu, welches aufgrund seines hohen Anteils an Ölsäure bei niedrigem Gehalt an mehrfach ungesättigten Fettsäuren als Einsatzstoff ebenfalls in Betracht kommen könnte. Für die Herstellung von hochungesättigten Fettalkoholen nach Verfahren 3 kommen schließlich Rapsöl, Olivenöl, Leinöl oder Erdnußöl in Frage. Fettalkohole auf dieser Basis enthalten jedoch einen signifikanten Anteil an mehrfach ungesättigten Homologen und sind daher anfällig gegenüber Autoxidationsprozessen.

Aus der deutschen Patentschrift **DE-C1 4422858** (Henkel) ist femer ein Verfahren bekannt, bei dem man Fettalkohole mit einem Trübungspunkt oberhalb 1°C durch Umesterung von Sonnenblumenöl bzw. LS ("low stearic") Sonnenblumenöl mit Methanol und anschließender Hydrierung der resultierenden Methylester herstellen kann. Aus anwendungstechnischen Gründen ist es jedoch häufig erforderlich, ungesättigte Fettalkohole einzusetzen, die einen noch niedrigeren Trübungspunkt aufweisen.

Demzufolge hat die Aufgabe der Erfindung darin bestanden, ein Verfahren zur Herstellung von autoxidationsstabilen, vorzugsweise pflanzlichen ungesättigten Fettalkoholen zur Verfügung zu stellen. die sich einerseits durch eine lodzahl oberhalb von 40, vorzugsweise von 80 bis 100 auszeichnen und gleichzeitig einen Trübungspunkt von maximal 0°C, vorzugsweise maximal -1°C und insbesondere maximal -2°C besitzen.

### Beschreibung der Erfindung

Gegenstand der Erfindung ist ein Verfahren zur Herstellung von ungesättigten Fettalkoholen mit verbessertem Kälteverhalten, bei dem man primäre Alkohole der Formel (I),

**R**^{**1**}**OH (I)**

in der R¹ für einen ungesättigten oder überwiegend ungesättigten Kohlenwasserstoffrest mit 6 bis 22 Kohlenstoffatomen steht, einer fraktionierten Kristallisation unterwirft.

Überraschenderweise wurde gefunden, daß auch stearinarme Fettalkohole mit hoher lodzahl und bereits verbessertem Kälteverhalten hinsichtlich des Trübungspunktes weiter optimiert werden können, wenn sie in geeigneter Weise einer fraktionierten Kristallisation unterworfen werden. Damit ist es möglich, Trübungspunkte unterhalb von 0°C und teilweise unter -3°C zu erzielen. Dies ist um so erstaunlicher, als der Festpunkt der Hauptkomponente Oleylalkohol 6 bis 7°C beträgt und in dem Gemisch zumindest vor der fraktionierten Kristallisation nennenswerte Anteile der höherschmelzenden Komponenten Stearylalkohol (Festpunkt 59°C) und Elaidylalkohol (Festpunkt 34 bis 37°C) enthalten sind. Die Erfindung schließt die Erkenntnis ein, daß der niedrige Trübungspunkt des durch fraktionierte Kristallisation hergestellten Alkohols nur aus einem bislang nicht bekannten überproportionalen Einfluß der in Spuren vorhandenen zweifach ungesättigten Alkohole resultiert, wobei nur die cis-cis-Komponenten einen Festpunkt unterhalb von 0°C aufweisen.

### Ungesättigte Fettalkohole

Primäre Alkohole, die im Sinne des erfindungsgemäßen Verfahrens als Einsatzstoffe dienen können, weisen 6 bis 22 und vorzugsweise 16 bis 18 Kohlenstoffatome auf. Beispiele für derartige Rohstoffe sind ungesättigte Fettalkohole wie etwa Palmoleylalkohol, Oleylalkohol, Elaidylalkohol, Petroselinylalkohol, Linolylalkohol, Linolenylalkohol, Ricinolalkohol, 12-Hydroxystearylalkohol, Gadoleylalkohol und Erucylalkohol. In der Praxis wird man jedoch seltener die reinen ungesättigten Fettalkohole, sondern technische Gemische mit gesättigten Homologen einsetzen, die eine lodzahl im Bereich von 50 bis 150, vorzugsweise 85 bis 98 aufweisen. Demzufolge ist der Begriff "überwiegend ungesättigt" so zu verstehen, daß mindestens 50 Mol-% des Fettalkoholgemisches ungesättigte Homologe enthält. die über 1, 2 oder 3 Doppelbindungen verfügen. Typische Beispiele für derartige Gemische sind tierische Fettalkohole wie beispielsweise Talgfettalkohol oder pflanzliche Fettalkohole wie beispielsweise Palmalkohol, Sonnenblumenfettalkohol, Rübalkohol und dergleichen. Die Fettalkohole lassen sich beispielsweise auf folgende Weisen herstellen:
(a) durch Spaltung von Triglyceriden, vorzugsweise Rindertalg oder Schweineschmalz, Anreicherung der ungesättigten Fettsäuren über den Weg der Umnetztrennung, Veresterung der Fettsäuren mit Methanol, Hydrierung der Methylester und gegebenenfalls destillative Aufarbeitung, oder
(b) durch Umesterung von Triglyceriden, vorzugsweise Rüböl, Sonnenblumenöl, Sojaöl, Erdnußöl und dergleichen mit Methanol, Hydrierung der Methylester und gegebenenfalls destillative Aufarbeitung.

Unter Umnetztrennung ist dabei das bekannte Verfahren zu verstehen, bei dem man Mischungen von gesättigten und ungesättigten Fettsäuren soweit abkühlt, daß die festen Anteile ausfallen, die resultierende Suspension mit einer wäßrigen Netzmittellösung behandelt und die entstehende Emulsion anschließend durch Zentrifugieren in einen Anteil mit niedriger und einen mit hoher lodzahl auftrennt [vgl. K.Schmid in **Fat Sci.Technol. 89, 237 (1987)].**

### Fraktionierte Kristallisation

Die fraktionierte Kristallisation stellt ein an sich bekanntes technisches Verfahren dar, welches beispielsweise bei der Reinigung von Ölen oder Spaltfettsäuren angewendet wird. Die Fraktionierung kann sowohl lösungsmittelfrei aus der Schmelze oder aber als Lösemittelkristallisation durchgeführt werden. Im letzten Fall wird der Fettalkohol üblicherweise in einer Konzentration von 5 bis 40 und vorzugsweise 10 bis 30 Gew.-% in einem geeigneten Solvens wie beispielsweise Aceton, Tetrahydrofuran, Methanol oder Ethanol gelöst und anschließend bei im Vergleich zur Schmelzkristatlisation niedrigeren Temperaturen fraktioniert. Der Prozeß der fraktionierten Kristallisation kann sowohl batchweise, wie z.B. in einem diskontinuierlichen Rührkessel, einem Fallfilmkristallisator oder anderen, sich belegenden Oberflächenkühlem als auch kontinuierlich, z.B. in Kristallisationskolonnen, mechanisch zwangsfördemden Apparaturen wie Kratzkühlern oder Kristallisierschnecken, oder aber in Sedimentationsapparaten wie Teller- oder Kühlscheibenkristallisatoren durchgeführt werden.

Vor der Abkühlung der ungesättigten Einsatzstoffe im Kristallisator ist es empfehlenswert, wenn auch nicht zwingend, den Fettalkohol mit Kristallen aus einer vorangegangenen Kristallisation zu impfen. Abhängig von der Qualität der eingesetzten Fettalkohole sollte die Fraktionierung bei Temperaturen im Bereich von 5 bis 35, vorzugsweise 10 bis 25°C starten, wobei die niedrigeren Temperaturen in der Regel nur für pflanzliche Fettalkohole mit hoher lodzahl Bedeutung haben. Die Abkühlung im Kristallisator sollte langsam erfolgen und in Abhängigkeit von Ausgangsgemisch und Lösemittel Haltepunkte aufweisen. Typische Abkühlgeschwindigkeiten liegen im Bereich von 1 bis 10, vorzugsweise 2 bis 5 K/h. Werden Fettalkohole mit niedrigem Erstarrungspunkt eingesetzt, kann bereits durch die Abtrennung einer Kristallfraktion eine Restschmelze mit einem Trübungspunkt unter 0°C erzielt werden. Vorzugsweise sollte die Fraktionierung jedoch mehrstufig, insbesondere in 2 bis 4 Stufen durchgeführt werden. Zur Steigerung der Selektivität ist es ferner möglich, eine der ersten Kristallfraktionen aufzuschmelzen, ein zweites Mal durch Kristallisation zu fraktionieren und die dabei erhaltene Restschmelze mit der Restschmelze des ursprünglichen Fraktionierschrittes zu vereinigen. Im Fall der fraktionierten Schmelzkristallisation empfiehlt es sich, den letzten Fraktionierschritt bei Temperaturen im Bereich von 1 bis 8, vorzugsweise 2 bis 5°C durchzuführen. Bei Einsatz von Lösemitteln wird die letzte Fraktionierung in der Regel bei Temperaturen im Bereich von -10 bis -20, vorzugsweise -12 bis -18°C durchgeführt.

### Gewerbliche Anwendbarkeit

Die nach dem erfindungsgemäßen Verfahren erhältlichen ungesättigten Fettalkohole weisen einen Trübungspunkt von 0°C und darunter auf. Sie eignen sich beispielsweise als Rohstoffe zur Herstellung von Tensiden und lassen sich durch Sulfatierung in Fettalkoholsulfate überführen. Als Co-Emulgatoren oder Konsistenzgeber können sie auch in kosmetische Zubereitungen eingearbeitet werden.

### Beispiele

### Beispiel 1:

Rindertalg einer technischen Qualität wurde in an sich bekannter Weise einer Druckspaltung, Umnetztrennung und katalytischen Hydrierung unterworfen. Auf diesem Wege wurde ein ungesättigter Talgfettalkohol mit einer lodzahl von 86 und einem Trübungspunkt von 9,5°C hergestellt. 500 g dieses Fettalkohols wurden in einem batchweise betriebenen 1-I-Rührkesselkristallisator überführt. Die Keimbildung wurde durch eine kurzzeitige Abkühlung des Ausgangsgemisches auf 5°C eingeleitet. Anschließend wurde das Gemisch auf 25°C erhitzt, mit einer Geschwindigkeit von 3 K/h auf 20°C abgekühlt und 2 h bei dieser Temperatur gehalten. Die entstandene Kristallsuspension wurde auf eine mit 20°C temperierte Filtemutsche aufgegeben und damit die Kristallfraktion F1 abgetrennt. Das Filtrat wurde in den Kristallisator zurückgeführt, wiederum mit 3 K/h auf 11°C abgekühlt und 2 h bei dieser Temperatur gehalten. Die Suspension wurde mit Hilfe der temperierten Nutsche getrennt und damit die Kristallfraktion F2 erhalten. Das Filtrat wurde abermals in den Kristallisator zurückgeführt, wiederum mit 3 K/h auf 6°C abgekühlt und 2 h bei dieser Temperatur gehalten. Die Suspension wurde mit Hilfe der temperierten Nutsche getrennt und damit die Kristallfraktion F3 sowie die Restschmelze F4 erhalten. In Tabelle 1 sind die Ausbeuten und die Kenndaten der Fraktionen zusammengefaßt.

**Tabelle 1**

| Fraktionierung von Fettalkohol (IZ = 86) | | | | |
|---|---|---|---|---|
| **Fraktion** | **Menge [g]** | **Ausbeute [%]** | **IZ** | **Trübungspunkt [°C]** |
| Ausgangsgemisch | 500 | | 86 | 9,5 |
| F1 | 10,5 | 2,1 | 60 | 30,7 |
| F2 | 29,0 | 5,8 | 69 | 23,9 |
| F3 | 148,5 | 29,7 | 81,9 | 14,1 |
| F4 | 312,5 | 62,4 | 92,1 | -1,6 |

### Beispiel 2

Analog Beispiel 1 wurde aus technischem Rindertalg ein ungesättigter Fettalkohol mit einer lodzahl von 91 und einem Trübungspunkt von 4,6°C hergestellt. Die Keimbildung wurde durch eine kurzzeitige Abkühlung des Ausgangsgemisches auf 1°C eingeleitet. Anschließend wurde das Gemisch auf 11°C erwärmt, mit einer Geschwindigkeit von 3 K/h auf 6°C abgekühlt und 2 h bei dieser Temperatur gehalten. Die entstandene Kristallsuspension wurde auf eine mit 11°C temperierte Filternutsche aufgegeben und damit die Kristallfraktion F1 abgetrennt. Das Filtrat wurde in den Kristallisator zurückgeführt, wiederum mit 3 K/h auf 3°C abgekühlt und 2 h bei dieser Temperatur gehalten. Die Suspension wurde mit Hilfe der temperierten Nutsche getrennt und damit die Kristallfraktion F2 erhalten. Das Filtrat wurde abermals in den Kristallisator zurückgeführt, wiederum mit 3 K/h auf 2°C abgekühlt und 2 h bei dieser Temperatur gehalten. Die Suspension wurde mit Hilfe der temperierten Nutsche getrennt und damit die Kristallfraktion F3 sowie die Restschmeize F4 erhalten. In Tabelle 2 sind die Ausbeuten und die Kenndaten der Fraktionen zusammengefaßt.

**Tabelle 2**

| Fraktionierung von Fettalkohol (IZ = 91) | | | | |
|---|---|---|---|---|
| **Fraktion** | **Menge [g]** | **Ausbeute [%]** | **IZ** | **Trübungspunkt [°C]** |
| Ausgangsgemisch | 500 | | 91 | 4,6 |
| F1 | 7,2 | 1,4 | 78 | 15,2 |
| F2 | 63,3 | 12,6 | 84 | 12,8 |
| F3 | 91,5 | 10,2 | 87 | 9,0 |
| F4 | 378,0 | 75,8 | 92 | -2,8 |

### Beispiel 3

Neues LS-Sonnenblumenöl mit einem Ölsäureanteil von mehr als 85 Gew.-% und einem Stearinsäureanteil von weniger als 3 Gew.-% wurde mit Methanol umgeestert und die resultierenden Methylester zu dem entsprechenden Fettalkohol mit einer lodzahl von 93 und einem Trübungspunkt von 1°C hydriert. 500 g dieses Fettalkohols wurden in einem batchweise betriebenen Fallfilmkristallisator, bestehend aus einem gekühlten, senkrecht stehenden Rohr mit einem temperierten Umwälzkreislauf eingefüllt. Die Keimbildung wurde durch kurzzeitige Abkühlung des Kühlmediums auf -5°C eingeleitet. Anschließend wurde das Gemisch mit einer Geschwindigkeit von 2 K/h auf 0°C abgekühlt und 1 h bei dieser Temperatur gehalten, wobei die Kristallfraktion F1 erhalten wurde. Die Restschmelze F2 wurde abgelassen. Anschließend wurde der Kristallisator auf 20°C aufgeheizt und dadurch die gebildete Fraktion F1 geschmolzen, so daß sie im flüssigen Zustand ablaufen konnte. Die Ergebnisse sind in Tabelle 3 zusammengefaßt.

**Tabelle 3**

| Fraktionierung von Fettalkohol (IZ = 93) | | | | |
|---|---|---|---|---|
| **Fraktion** | **Menge [g]** | **Ausbeute [%]** | **IZ** | **Trübungspunkt [°C]** |
| Ausgangsgemisch | 500 | | 93 | 1.0 |
| F1 | 158 | 31,6 | 90 | 7,2 |
| F2 | 342 | 68,4 | 93 | -3,8 |

## Patentansprüche

1. Verfahren zur Herstellung von ungesättigten Fettalkoholen mit verbessertem Kälteverhalten, **dadurch gekennzeichnet, daß** man primäre Alkohole der Formel (I),
**R**^{**1**}**OH (I)**
in der R¹ für einen ungesättigten oder überwiegend ungesättigten Kohlenwasserstoffrest mit 6 bis 22 Kohlenstoffatomen steht, einer fraktionierten Kristallisation unterwirft.

2. Verfahren nach Anspruch 1, **dadurch gekennzeichnet, daß** man technische Fettalkoholgemische einsetzt, die eine lodzahl im Bereich von 50 bis 150 aufweisen.

3. Verfahren nach den Ansprüchen 1 und 2, **dadurch gekennzeichnet, daß** man Fettalkohole einsetzt, die man
(a) durch Spaltung von Triglyceriden, Anreicherung der ungesättigten Fettsäuren über den Weg der Umnetztrennung, Veresterung der Fettsäuren mit Methanol, Hydrierung der Methylester und gegebenenfalls destillative Aufarbeitung, oder
(b) durch Umesterung von Triglyceriden mit Methanol, Hydrierung der Methylester und gegebenenfalls destillative Aufarbeitung
erhält.

4. Verfahren nach den Ansprüchen 1 bis 3, **dadurch gekennzeichnet, daß** man die fraktionierte Kristallisation aus der Schmelze oder in einem Lösungsmittel durchführt.

5. Verfahren nach den Ansprüchen 1 bis 4, **dadurch gekennzeichnet, daß** man die fraktionierte Kristallisation in Bauteilen durchführt, die ausgewählt sind aus der Gruppe der diskontinuierlichen Rührkessel, Fallfilmkristallisatoren, Kristallisationskolonnen, Kratzkühler, Kristallisierschnecken, Teller- oder Kühlscheibenkristallisatoren.

6. Verfahren nach den Ansprüchen 1 bis 5, **dadurch gekennzeichnet, daß** man die Fraktionierung in Gegenwart von Impfkristallen aus einer vorangegangenen Kristallisation durchführt.

7. Verfahren nach den Ansprüchen 1 bis 6, **dadurch gekennzeichnet, daß** man die Fraktionierung mit einer Geschwindigkeit von 1 bis 10 K/h durchführt.

8. Verfahren nach den Ansprüchen 1 bis 7, **dadurch gekennzeichnet, daß** man die fraktionierte Kristallisation in zwei bis vier Stufen durchführt.

9. Verfahren nach den Ansprüchen 1 bis 8, **dadurch gekennzeichnet, daß** man die erhaltenen Kristallfraktionen aufschmilzt, ein zweites Mal durch Kristallisation fraktioniert und die dabei erhaltene Restschmelze mit der Restschmelze des ursprünglichen Fraktionierschrittes vermischt.

10. Verfahren nach den Ansprüchen 1 bis 9, **dadurch gekennzeichnet, daß** man den letzten Fraktionierschritt bei Temperaturen im Bereich von 1 bis 8°C durchführt.

## Claims

1. A process for the production of unsaturated fatty alcohols with improved low-temperature behavior, **characterized in that** primary alcohols corresponding to formula (I):
**R**^{**1**}**OH (I)**
in which R¹ is an unsaturated or predominantly unsaturated hydrocarbon radical containing 6 to 22 carbon atoms, are subjected to fractional crystallization.

2. A process as claimed in claim 1, **characterized in that** technical fatty alcohol mixtures with an iodine value of 50 to 150 are used.

3. A process as claimed in claims 1 and 2, **characterized in that** fatty alcohols obtained by
(a) splitting of triglycerides, enrichment of the unsaturated fatty acids by rolling-up separation, esterification of the fatty acids with methanol, hydrogenation of the methyl esters and optionally working up by distillation,
(b) transesterification of triglycerides with methanol, hydrogenation of the methyl esters and optionally working up by distillation are used.

4. A process as claimed in claims 1 to 3, **characterized in that** the fractional crystallization is carried out from the melt or in a solvent.

5. A process as claimed in claims 1 to 4, **characterized in that** the fractional crystallization is carried out in units selected from the group consisting of discontinuous stirred tank reactors, falling-film crystallizers, crystallization columns, scraper condensers, crystallization screws, plate or cooling disk crystallizers.

6. A process as claimed in claims 1 to 5, **characterized in that** the fractionation is carried out in the presence of seed crystals from a previous crystallization.

7. A process as claimed in claims 1 to 6, **characterized in that** the fractionation is carried out at a rate of 1 to 10 K/h.

8. A process as claimed in claims 1 to 7, **characterized in that** the fractional crystallization is carried out in two to four stages.

9. A process as claimed in claims 1 to 8, **characterized in that** the crystal fractions obtained are melted, fractionated by crystallization a second time and the residual melt obtained is mixed with the residual melt of the original fractionation step.

10. A process as claimed in claims 1 to 9, **characterized in that** the last fractionation step is carried out at temperatures of 1 to 8°C.

## Revendications

1. Procédé de préparation d'alcools gras non saturés ayant des propriétés au froid améliorées, **caractérisé en ce que** on soumet des alcools primaires de formule (I) :
R¹ OH (I)
dans laquelle R¹ représente un reste hydro carboné non saturé ou non saturé d'une manière prédominante, ayant de 6 à 22 atomes de carbone, à une cristallisation fractionnée.

2. Procédé selon la revendication 1, **caractérisé en ce que** on met en oeuvre des mélanges industriels d'alcools gras qui possèdent un indice d'iode dans la zone de 50 à 150.

3. Procédé selon les revendications 1 et 2, **caractérisé en ce que** on met en oeuvre des alcools gras que l'on obtient :
a) par coupure de triglycérides, enrichissement des acides gras non saturés par le moyen de la séparation par inversion de phase, estérification des acides gras avec du méthanol, hydrogénation de l'ester méthylique et le cas échéant purification par distillation, ou
b) par transestérification des triglycérides avec des méthanol, hydrogénation de l'ester méthylique et le cas échéant purification par distillation.

4. Procédé selon les revendications 1 à 3, **caractérisé en ce que** on effectue la cristallisation fractionnée à partir de produit de fusion ou dans un solvant.

5. Procédé selon les revendications 1 à 4, **caractérisé en ce que** on effectue la cristallisation fractionnée dans des éléments de construction qui sont choisis dans le groupe de la chaudière à agitation en discontinu, des cristallisations à film tombant, des colonnes de cristallisation, des échangeurs de chaleur à raclage, des vis sans fin de cristalliseur, des cristalliseurs en assiette ou en disque de réfrigération.

6. Procédé selon les revendications 1 à 5, **caractérisé en ce que** on effectue le fractionnement en présence de cristaux d'ensemencement provenant d'une cristallisation précédente.

7. Procédé selon les revendications 1 à 6, **caractérisé en ce que** on effectue le fractionnement à une vitesse de 1 à 10 K/h.

8. Procédé selon les revendications 1 à 7, **caractérisé en ce que** on effectue la cristallisation fractionnée en deux à quatre étapes.

9. Procédé selon les revendications 1 à 8, **caractérisé en ce qu'** on fait fondre les fractions de cristaux obtenus, on fractionne une deuxième fois par cristallisation et les produits de fusion résiduels ainsi obtenus sont mélangés avec les produits de fusion résiduels de l'étape de fractionnement d'origine.

10. Procédé selon les revendications 1 à 9, **caractérisé en ce que** on effectue la dernière étape de fractionnement à des températures dans la zone de 1 à 8°C.
